Europäisches Patentamt

European Patent Office

Office européen des brevets

(11) Publication number: **0 280 509**
A1

# (12) EUROPEAN PATENT APPLICATION

(21) Application number: 88301531.5

(22) Date of filing: 23.02.88

(51) Int. Cl.⁴: **A 61 F 5/01**

(30) Priority: 23.02.87 GB 8704168

(43) Date of publication of application:
31.08.88 Bulletin 88/35

(84) Designated Contracting States:
AT BE CH DE ES FR IT LI NL SE

(71) Applicant: JOHNSON & JOHNSON ORTHOPAEDICS INC.
501 George Street
New Brunswick New Jersey 08903 (US)

(72) Inventor: Adams, Gwyneth
26 Windsor Street
Colne Lancashire (GB)

(74) Representative: Fisher, Adrian John et al
CARPMAELS & RANSFORD 43 Bloomsbury Square
London WC1A 2RA (GB)

(54) A cervical collar and cover.

(57) A cervical collar is provided which comprises an elongate flexible container, such as stockinette, containing particulate material, such as expanded polystyrene. The ends of the collar are provided with fastening means for securing the collar around the neck of a patient. The container may be divided into two or more longitudinally extending compartments.

The fastening means may be provided on a removable cover for the collar.

Fig.8

EP 0 280 509 A1

**Description**

## A CERVICAL COLLAR AND COVER

This invention relates to a cervical collar and a cover for such a collar.

It has been proposed in British Patent Specification No. 1422966 to form a body splint from an air-tight flexible bag containing pellets, granules or the like of expanded polystyrene. The air-tight flexible bag is evacuated to cause the pellets, granules or the like to come into contact and eliminate their freedom of movement within the bag, thereby to form a rigid moulding. It is apparent that such a splint would have to applied by a trained technician, with the use of specialised evacuating equipment.

British Patent Specification No. 1456831 discloses a temporary splint formed from a filled gas-impervious container with thin deformable walls having sealed within it both a body of gas-absorbent particulate material and an amount of gas absorbable thereby. At a first, higher temperature the gas is at least partially unabsorbed into the particles, so that the particles are loose within the container and the overall shape of the container is readily deformable to any desired shape. At a second, lower temperature, substantially all of the gas is absorbed and the deformable walls are thus drawn in upon the body of material to rigidify the container and contents in the desired shape.

Common to these two prior proposals is the concept of a rigid splint formed from a gas-impervious, partially evacuated flexible container filled with particulate material. In contrast, the present invention provides a cervical collar comprising an elongate flexible container containing a particulate material, the ends of the collar being provided with fastening means for securing the collar around the neck of a patient.

It has been found that the cervical collar according to the invention, by virtue of being formed into a loop around the neck of a patient by the fastening means, provides sufficient support without any need to render it rigid by partial evacuation. This greatly simplifies the manufacture of the collar and this renders it very economical to produce.

Preferably, the container is made of material which is elastic in nature and is filled to such a degree that the particles are in close contact and cause the material of the container to stretch. It is particularly preferred that the container be formed from a mesh-like material such as stockinette.

The particulate material preferably has a bulk density of from 1 to 1000g/dm³, and more preferably from 5 to 100g/dm³. Particulate material having a bulk density of from 10 to 20g/dm³ is especially preferred.

The particles of the particulate material will usually have an average diameter of from 0.5 to 15mm, and preferably from 1 to 8mm. A particle size of from 3 to 5mm is most preferably employed.

The particulate matter is preferably in the form of lightweight plastic beads such as expanded polystyrene beads.

The container is preferably divided into two or more longitudinally extending compartments such as by a line of sewing. By this means, the collar can be well filled with particulate material without making it unduly thick in relation to its width. Conveniently, the collar is divided into three or more such compartments by two or more lines of sewing. The central compartment or compartments may contain more particulate material than the outer compartments for improved support of the neck

The fastening means for fastening the collar in a wrapped-around manner around a patient's neck may be provided on a removable cover for the collar. The fastening means may be of a wide variety of types, but a fabric hook and loop fastener, such as is sold under the Trade Mark VELCRO, is especially preferred.

One or more projecting holding means may be provided on which the patient may pull to rotate the collar around the neck, or to aid fastening and removing the collar. The projecting holding means are preferably provided on a removable cover for the collar.

The collar according to the invention is particularly useful to a patient who is unable to tolerate more standard, soft collars, such as may be made of polyurethane foam, or polyethylene foam for example patients suffering from motor neurone desease, cervical spondylosis, ankylosing spondylitis or rheumatoid arthritis.

A preferred embodiment of the invention will now be described, by way of example, with reference to the accompanying drawings, in which:

    Figures 1 to 5 show a stockinette in various stages of preparation of the cervical collar; and

    Figures 6 to 8 show another stockinette in various stages of preparation of a cover for the cervical collar.

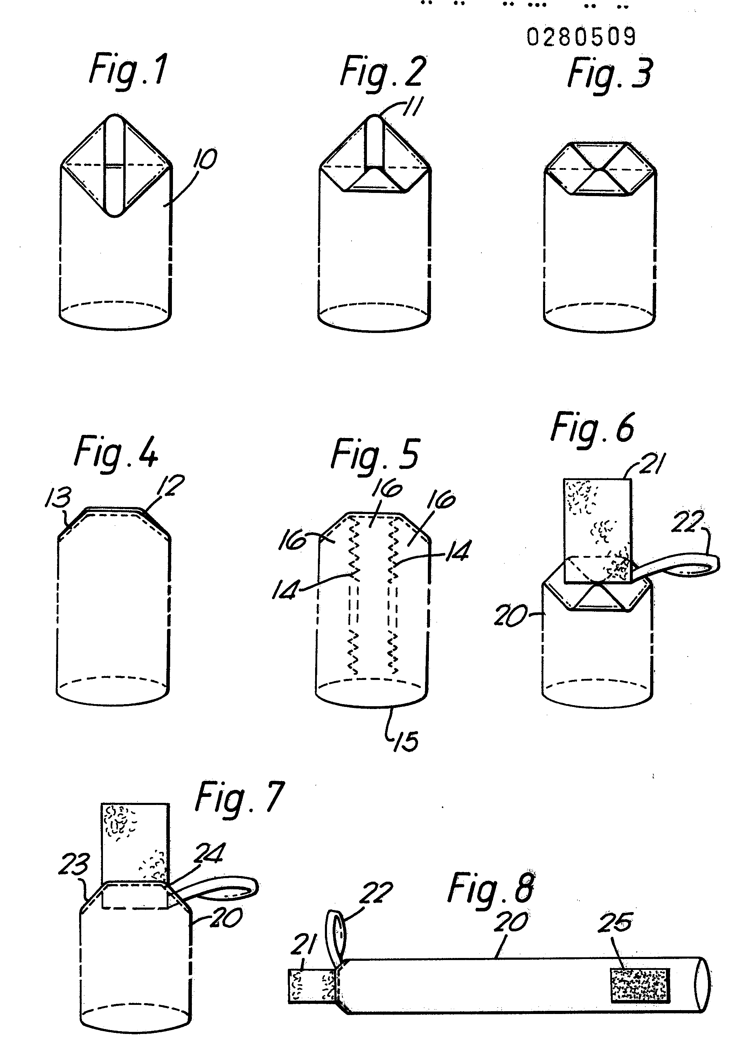

The cervical collar of the preferred embodiment is made as follows. A piece of stockinette 10, 8cm in width and between 50 and 55cm in length, is laid out flat and the two corners at one end are folded inwards, as shown in Figure 1. The extremities 11 of the two flaps thus formed are folded inwards, towards each other, as shown in Figures 2 and 3, and the two flaps are folded together, as shown in Figure 4. The chamfered end 12 of the stockinette 10, thus formed, is sewn up along edge 13, forming a bag.

At approximately 2 1/2 cm in from the edges of the bag, two lines of zig-zag stitching 14 are formed, parallel to the edges of the bag, through the double thickness of material provided by the flattened stockinette. The two lines of stitching 14 end approximately 3cm from the end 15 of the stockinette, remote from the chamfered end 12. In this manner, three tubes 16 are formed in the stockinette.

Each of the two outer tubes 16 is filled with approximately 0.2 l of polystyrene beads, using a funnel to prevent spillage. The two outer tubes are temporarily closed by means of a pin. The remaining middle tube is now filled with approximately 0.3 to 0.4 l of polystyrene beads and is secured with a pin. The end 15 of the stockinette is now folded and sewn up, in the same manner as the end 12.

The lines of zig-zag stitching 14 are continued to the new chamfered end and the pins are removed. The cervical collar is now complete, and a gentle shaking will evenly distribute the beads inside.

A cover 20 for the stockinette is formed as follows, with reference to Figures 6 to 8. A second piece of stockinette, of 8cm width and between 52 and 57cm length, is laid out flat and one end is folded as shown in Figures 1 to 3. A hook 21 in the form of a VELCRO strip 5cm wide, is placed between the flaps formed by the folded end of the stockinette. A piece of tape 1.3 cm wide and 13cm long is folded double and the two ends thereof are placed beside the hook 21, to form a loop 22 (Figures 6) . The end 23 of the stockinette 20 is now sewn up along stitch line 24 (Figure 7) so as to secure the hook 31 and loop 22 in the sewn-up end. A VELCRO tab 25, 5cm in width, is sewn onto one side of the stockinette 20, with the end of the tab 25 being aproximately 6cm from the end of the stockinette 20. The cover so formed is now complete, as shown in Figure 8.

To use the invention in its preferred embodiment, the collar, made as described, is inserted in the open end of the cover 20 and the open end is tucked in, to completely enclose the collar. The collar and cover are then wrapped around the patient's neck and the hook 21 is stuck to the tab 25 to hold the collar and cover on the patient. Because of the uniform contour of the collar along its length. it can be fastened to the neck from the front. A patient will thus find it possible to put on the collar, with very little dexterity required, and the collar and cover can then be rotated around the neck, to a desired position, by pulling with the thumbs, finger or mechanical aid on the loop 22. This feature is particularly important to patients suffering from rheumatoid arthritis Once the collar is positioned around the patient's neck, tension in the outer part of the stockinette 10 forces the polystyrene beads together, which then gently immobilise the neck. The beads can be manually moved circumferentially to fit the contours of the jaw line and larynx for improved patient comfort.

The widths of the longitudinal tubes 16 can be varied to allow for contouring around the neck.

The cover, which, of course, is removable, allows for ease of fastening and for hygiene. The collar could be used without such a cover, and the fastening means could be provided on the collar itself.

It will, of course, be understood, that the above description has been given by way of example only, and that modifications of detail can be made within the scope of the invention.

## Claims

1. A cervical collar comprising an elongate flexible container (10, 20) containing a particulate material, the ends of the collar being provided with fastening means (21) for securing the collar around the neck of a patient.

2. A collar according to claim 1, wherein the container is made of material which is elastic in nature and is filled to such a degree that the particles are in close contact and cause the material of the container to stretch.

3. A collar according to either of claims 1 and 2 wherein the container is formed from a mesh-like material such as stockinette.

4. A collar according to any one of claims 1 to 3, wherein the particulate material has a bulk density of from 1 to 1000g/dm$^3$.

5. A collar according to any one of the preceding claims wherein the particles of the particulate material have an average diameter of from 0.5 to 15mm.

6. A collar according to any one of the preceding claims, wherein the container is divided into two or more longitudinally extending compartments (16).

7. A collar according to claim 6 comprising one or more central longitudinally extending compartment or compartments flanked by two outer longitudinally extending compartments, wherein the or each central longitudinally extending compartment contains more particulate material than the outer longitudinally extending compartments.

8. A collar according to any one of the preceding claims, wherein the fastening means is a fabric hook and loop fastener.

9. A collar according to any one of the preceding claims wherein the fastening means are provided on a removable cover (20) for the collar (10).

10. A collar according to any one of the preceding aims together with one or more projecting holding means (22) for pulling to rotate the collar around the neck.

Fig.1    Fig.2    Fig.3

0280509

Fig.4    Fig.5    Fig.6

Fig.7    Fig.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 374 785 (GAYLORD)<br>* claims 1,2; figures * | 1,6,8 | A 61 F 5/01 |
| A | DE-U-8 318 991 (Dr. P. KOCH KG)<br>* claim 1; page 5, lines 13-20 * | 1,6-8 | |
| A | US-A-3 745 998 (ROSE)<br>* claim 1; figure 13 * | 1,8 | |
| D,A | GB-A-1 422 966 (SCHETRUMPF)<br>* claims 1,3 * | 1 | |
| D,A | GB-A-1 456 831 (T.F. SMITH & NEPHEW LTD.)<br>* page 1, lines 9-19; claim 1 * | 1 | |

TECHNICAL FIELDS
SEARCHED (Int. Cl.4)

A 61 F 5/00
A 61 F 13/00

The present search report has been drawn up for all claims

| Place of search | Date of completion of the search | Examiner |
|---|---|---|
| BERLIN | 13-05-1988 | KANAL P K |

EPO FORM 1503 03.82 (P0401)